Europäisches Patentamt

European Patent Office

Office européen des brevets

(11) Numéro de publication: **0 412 877 A1**

## (12) DEMANDE DE BREVET EUROPEEN

(21) Numéro de dépôt: 90402199.5

(22) Date de dépôt: 01.08.90

(51) Int. Cl.⁵: **A61K 9/20**, A61K 47/42, A61K 31/44

(30) Priorité: 07.08.89 FR 8910605

(43) Date de publication de la demande:
**13.02.91 Bulletin 91/07**

(84) Etats contractants désignés:
**AT BE CH DE DK ES FR GB GR IT LI LU NL SE**

(71) Demandeur: **Laboratoires DELAGRANGE**
**1, Avenue Pierre-Brossolette**
**F-91380 Chilly-Mazarin(FR)**

(72) Inventeur: **Besancon, Denis, Dr.**
**31 bld. de Latour-Maubourg**
**F-75007 Paris(FR)**
Inventeur: **Aiache, Jean-Marc, Pr.**
**17, rue du Maréchal Galiéni**
**F-63000 Clermont-Ferrand(FR)**
Inventeur: **Dufour, Alain**
**42, avenue de Saxe**
**F-75007 Paris(FR)**
Inventeur: **Egros, Fabrice**
**3, Avenue de la Résidence**
**F-92160 Anthony(FR)**

(54) **Nouvelle forme galénique orale améliorant la biodisponibilité.**

(57) L'invention concerne une nouvelle forme galénique améliorant la biodisponibilité d'un médicament.
L'insertion de protéines naturelles dans la formule entraîne une libération contrôlée du principe actif. Elle permet l'obtention de formes unidoses applicables notamment aux dihydropyridines possèdant des propriétés antagonistes du calcium. Ces formes particulières sont utiles pour réaliser une prise thérapeutique quotidienne.

EP 0 412 877 A1

## NOUVELLE FORME GALENIQUE ORALE AMELIORANT LA BIODISPONIBILITE

L'invention concerne une forme galénique nouvelle, permettant de maîtriser la biodisponibilité de médicaments, et en particulier de ceux qui constituent la classe des inhibiteurs calciques.

Parmi ceux-ci, des dérivés de dihydropyridine doués de précieuses propriétés cardiovasculaires voient leur utilisation limitée par des problèmes de dissolution in vivo, donc en milieu aqueux.

La demanderesse a donc cherché à obtenir une forme galénique particulière, pour adapter la libération in vivo de tels principes actifs à l'obtention de taux plasmatiques suffisants pour une prise quotidienne, quelle que soit la solubilité de ces principes actifs.

L'essentiel de l'invention réside dans le choix d'un substrat protéique spécifique permettant d'atteindre cet objectif.

Il est connu d'associer un principe actif à un substrat cristallin soluble dans l'eau, tel que le polyéthylèneglycol ou la polyvinylpyrrolidone, sous forme d'agent dispersant, pour obtenir des formes retard, comme il est décrit par exemple dans le brevet français 2.565.822. Mais la dispersion qui en résulte, transformée en comprimés ou gélules avec des excipients classiques, subit, comme cela est indiqué dans le brevet précité, "une décharge rapide du médicament pendant le passage dans l'estomac", à moins d'être protégée par un ester glycérique d'acide gras, ou par un enrobage entérique, pour une absorption gastrointestinale. Cette décharge rapide entraîne généralement des effets secondaires tels que céphalées, rougeurs... etc...

L'expérience a démontré que l'obtention de ces dispersions, dans des conditions physico-chimiques connues conduisait généralement à un produit sous forme de blocs solides, difficilement broyables. Le broyage entraînant un effet de chauffage, le principe actif devient instable, et risque au minimum une décomposition partielle. Dans les autres cas, on obtient des substances collantes, difficiles à récupérer, et donc peu exploitables industriellement.("Stability Problems under special consideration of solid dispersion of drugs", S.T.P. Pharma 1 (7) 660-665 (1985)).

Les nombreuses solutions proposées dans la littérature n'avaient pas, à ce jour, réussi à satisfaire aux conditions d'absorption capables d'offrir une couverture immédiate après l'ingestion du médicament, et de se poursuivre régulièrement sur 24 heures.

L'objet de cette invention est de proposer une forme orale de tels médicaments, offrant une couverture constante sur 24 heures avec une seule prise quotidienne, y compris lorsque ces composés sont peu ou pas solubles dans l'eau. La maîtrise de la biodisponibilité implique, selon le cas, de ralentir ou accélérer la diffusion, mais surtout, de la rendre régulière.

L'exemple suivant en illustre l'intérêt pratique.

L'oxodipine (ou 1,4-dihydro 4-(2′,3′-méthylènedioxyphényl) 3,5-pyridine dicarboxylate de méthyle et d'éthyle) décrite dans le brevet français n° 2.562.069 a révélé une activité inhibitrice calcique puissante, susceptible d'application dans le traitement des troubles cardiovasculaires tels que l'hypertension. Cependant, sa faible solubilité dans l'eau semblait limiter ses possibilités d'utilisation dans des compositions pharmaceutiques.

Or, l'emploi de moyens usuels de solubilisation, tels que des solvants non aqueux (ethanol par exemple) ou l'adjonction de mouillants n'est pas toujours compatible avec une administration in vivo.

L'étude de la cinétique de dissolution in vitro, réalisée d'une part dans l'eau distillée et d'autre part dans du suc intestinal, montre que l'on obtenait déjà une amélioration, en utilisant un coprécipité oxodipine-polyvinylpyrrolidone, par rapport à l'oxodipine pure dont la dissolution est lente et limitée, ce qui se traduit par une absorption lente et limitée in vivo.

Mais cette dispersion solide présente les insuffisances déjà soulignées dans l'art connu, c'est-à-dire qu'elle libère de façon très rapide et intense la presque totalité du principe actif, ce qui ne permet pas une bonne maîtrise de la biodisponibilité sur une journée.

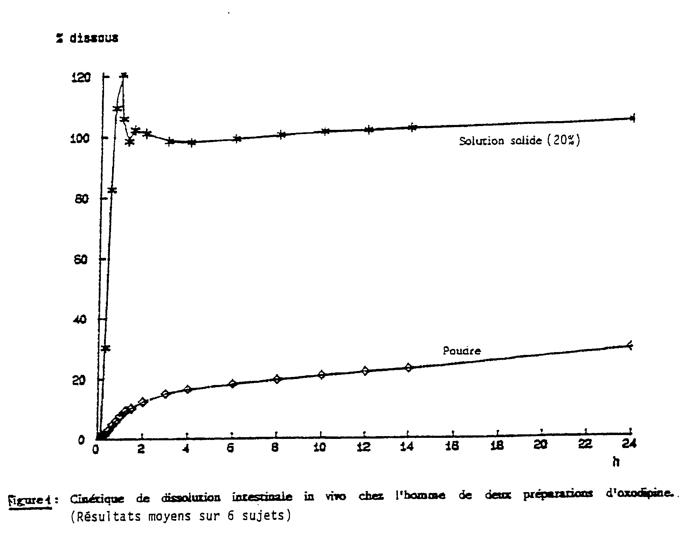

La figure 1 montre la cinétique de dissolution intestinale in vivo chez l'homme, de deux préparations d'oxodipine administrées par voie orale : "poudre" d'oxodipine pure, et "solution solide" d'oxodipine dans la polyvinylpyrrolidone, par rapport à une solution hydro alcoolique orale de référence, donnant 100% d'absorption, chaque forme étant dosée à 20 mg de principe actif. On voit que la "poudre" libère au maximum 20% des 20 mg administrés tandis que la "solution solide" en libère 100% qui sont totalement absorbés, presque immédiatement.

La demanderesse a découvert, en poursuivant ses recherches, que l'association d'une solution solide (d'oxodipine avec la polyvinylpyrrolidone par exemple) et de protéines permettait l'obtention d'un système thérapeutique satisfaisant, assurant le contrôle de l'absorption et de la biodisponibilité. Parmi ces protéines, deux ont été spécifiquement distinguées car elles permettent d'atteindre, au sein de cette association, les objectifs suivants :
- mettre en solution les insolubles
- contrôler la libération des solubles
- mettre à la disposition de l'organisme une dose rapide (de charge) et une dose contrôlée
- améliorer la dissolution, l'absorption et la biodisponibilité in vivo, chez l'homme.

Ces protéines naturelles sont extraites du lait écrémé frais et sont commercialisées sous l'appellation PROSOBEL - L 85 et PROSOBEL - L 60.

Des comprimés et des gélules ont été réalisés, avec l'oxodipine ou d'autres dihydropyridines comme principe actif. Puis, ils ont été administrés oralement à des volontaires sains; les taux plasmatiques de principe actif ont été contrôlés à intervalles réguliers.

Exemple 1

Préparation d'un co-précipité oxodipine - polyvinyl pyrrolidone

Dans un réacteur de 20 litres on a introduit 5800 ml d'éthanol absolu puis, par portions, en agitant, 2600 g de polyvinylpyrrolidone. On a chauffé jusqu'à environ 30°C pour tout dissoudre et ajouté 650 g d'oxodipine. On a chauffé doucement jusqu'à environ 80°C de façon à obtenir une solution totale et laissé reposer.

Quelques cristaux sont réapparus sur les parois du ballon. On a ajouté 1 litre d'éthanol et porté à nouveau à 80°C pour dissoudre le produit. On a laissé reposer 24 heures à température ambiante (environ 20°C) puis la solution a été évaporée. Le produit obtenu a été séché plusieurs heures à 80°C et broyé puis tamisé sur tamis de 400µ.

Le dosage par spectrométrie UV a donné les résultats suivants :
(360nm) OXODIPINE : 19,9%
PVP : 77,6%
Ethanol résiduel 1200 ppm

Exemple 2

Préparation de gélules à 10 mg d'Oxodipine

Dans la cuve d'un mélangeur "Turbula", on introduit les composants (A) et on homogénéise pendant 10 minutes.
On mélange avec les composants (B) lubrifiants, et on emplit des gélules n° 1 de ce mélange.

| Gélules références F | |
|---|---|
| (A) Oxodipine coprécipité (à 20% soit 10 mg d'oxodipine) | 0,050g |
| H P M C 15000 (hydroxy-propylmethyl cellulose) | 0,018g |
| PROSOBEL - L 60 | 0,113g |
| AMIDON DE MAIS | 0,014g |
| LACTOSE | 0,032g |
| | 0,227g |
| (B) TALC (2301 - ZA) | 0,0022g |
| Stéarate de Mg (2493-SW) | 0,0022g |
| Pour une gélule contenant 0,2314g | |

Gélule référence G

3

EP 0 412 877 A1

On reprend les mêmes quantités et les mêmes composants, mais le PROSOBEL L 60 est remplacé par du PROSOBEL - L 85.

Exemple 3

On réalise un comprimé unique à double couche, constitué par l'association physique (collage) de deux "sous-comprimés" (C), à libération rapide et (D) à libération lente, contenant le principe actif associé à des excipients usuels, et à du PROSOBEL - L85 ou L60 (qui donnent des résultats similaires).

L'ensemble constitue un système thérapeutique qui permet la libération contrôlée des doses de charge et d'entretien.

Les proportions préférées de ces sous-comprimés sont

| sous-comprimé C (en pourcentage de poids) | |
|---|---|
| Coprécipité d'oxodipine | 23,4 |
| ELCEMA - G250 | 70,1 |
| PRIMOJEL (carboxyméthylamidon) | 6,5 |
| | 100 |

| sous-comprimé D (en pourcentage de poids) | |
|---|---|
| Coprécipité d'oxodipine | 22 |
| PROSOBEL (L85 ou L60) | 50 |
| H P M C | 8 |
| Amidon | 6 |
| Lactose | 4 |
| COMPRITOL | 8 |
| Talc | 1 |
| Stéarate de Mg | 1 |
| | 100 |

A titre d'exemple, on dose le sous-comprimé C à 5mg d'oxodipine et le sous-comprimé D à 10,15, 20mg d'oxidipine, ce qui permet de disposer de dosages unitaires contenant 15, 20, et 25mg d'oxodipine.

Les formes pharmaceutiques nouvelles selon l'invention ont fait l'objet d'études in vitro et in vivo.

La figure 1 exprime, pour une même dose, le pourcentage de principe actif absorbé dans l'organisme humain (suc intestinal), pour l'oxodipine pure (poudre) et en solution solide de coprécipité oxodipine-polyvinylpyrrolidone (contenant 20% d'oxodipine). On voit que dans le cas de la solution solide, il y a libération immédiate et massive d'oxodipine (ce qui entraîne les réactions secondaires déjà décrites) tandis que dans le cas de l'oxodipine pure, on ne dépasse pas 20% d'absorption, ce qui est insuffisant.

Les gélules F et G (selon l'exemple 2) ont été administrées à 6 volontaires sains, et les taux plasmatiques d'oxodipine ainsi que les pourcentages d'oxodipine absorbés ont été suivis tout le long du nycthémère. Les résultats ont été comparés à ceux qui ont été obtenus avec les formes connues (solution hydroalcoolique, coprécipité, poudre d'oxodipine pure).

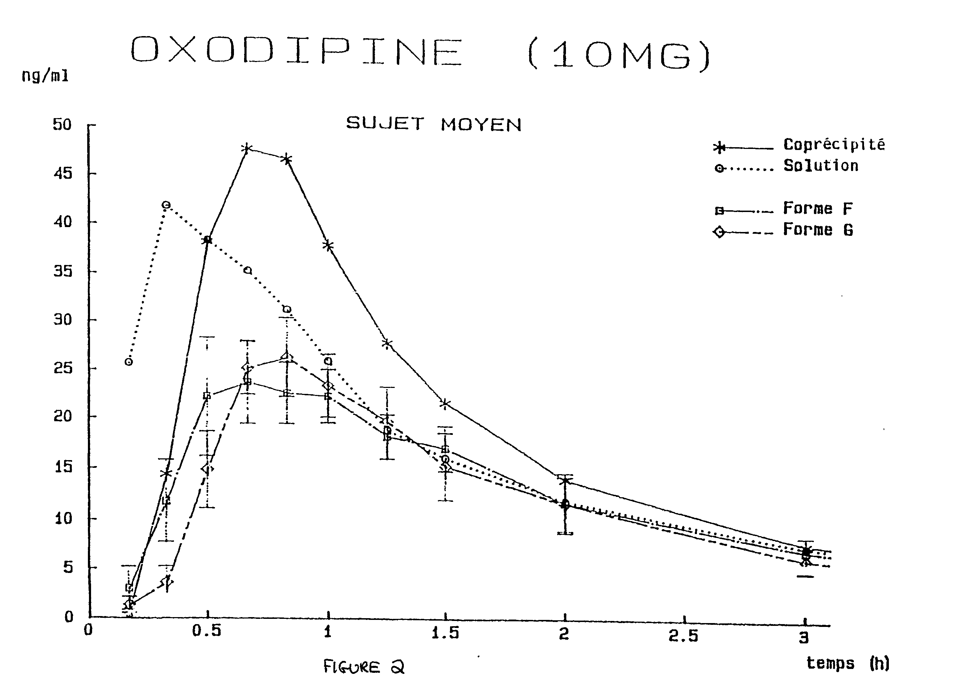

La figure 2 présente les résultats éclatés à 3 heures, et met en évidence des concentrations maximales réduites de moitié par rapport au coprécipité.

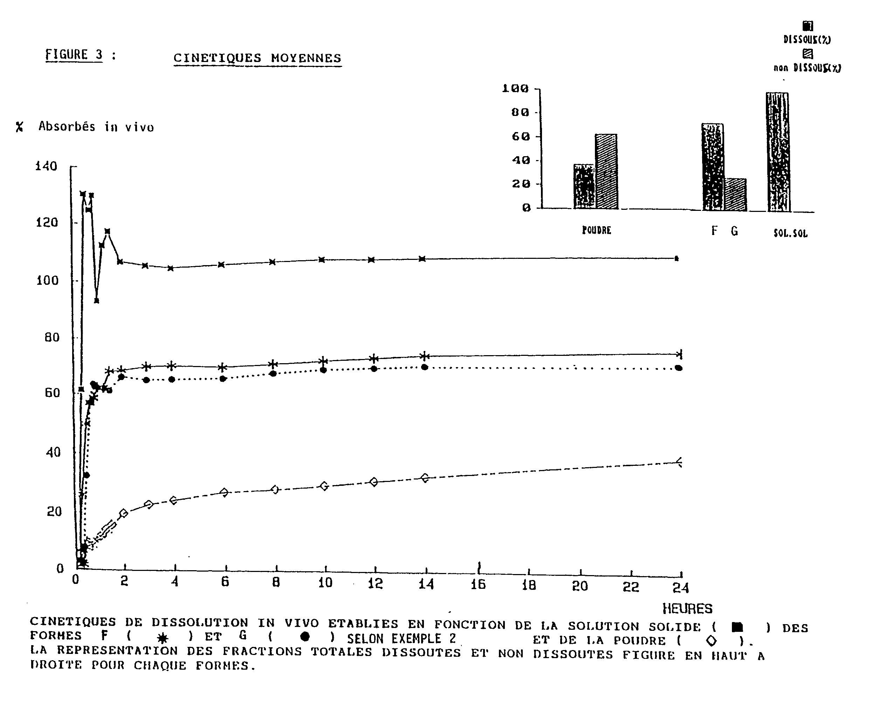

La figure 3 montre que les pourcentages absorbés à partir des gélules contenant du PROSOBEL sont intermédiaires entre une absorption trop rapide (coprécipité) ou trop lente (poudre), preuve d'un meilleur contrôle de l'absorption, avec le PROSOBEL.

D'autre part, on voit que la variété de PROSOBEL choisie (L60 ou L85) n'influe pas sur le résultat in vivo, chez l'homme.

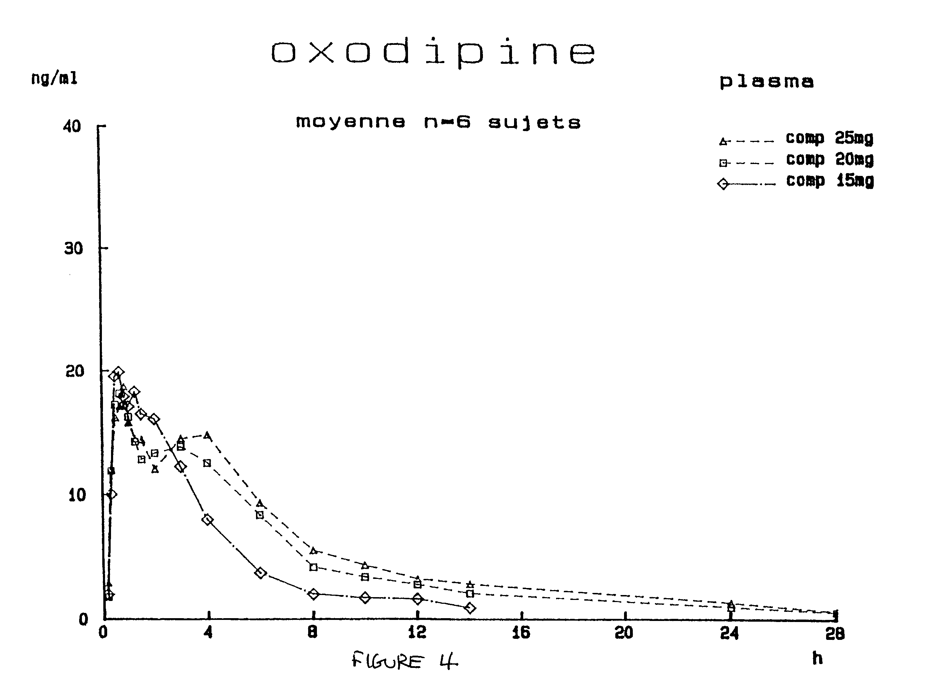

Des comprimés selon l'exemple 3, dosés à 15, 20 et 25mg d'oxodipine, ont été administrés à 6 volontaires sains. La figure 4 et le tableau 1 représentent l'évolution des concentrations plasmatiques, suivie

4

tout le long du nycthémère. Les résultats sont comparés à ceux que l'on obtient après administration de la poudre et du coprécipité par voie orale.

Tableau I

|  | Comprimé 15 mg | Comprimé 20 mg | Comprimé 25 mg |
|---|---|---|---|
| Temps (h) | Concentration en ng/ml | Concentration en ng/ml | Concentration en ng/ml |
| 0,00 | 0,0 | 0,0 | 0,0 |
| 0,17 | 2,0 | 1,8 | 2,9 |
| 0,33 | 10,0 | 11,9 | 12,0 |
| 0,50 | 19,5 | 17,2 | 16,1 |
| 0,66 | 19,9 | 18,1 | 17,0 |
| 0,83 | 17,8 | 17,1 | 18,6 |
| 1,00 | 17,0 | 16,2 | 15,7 |
| 1,25 | 18,2 | 14,2 | 14,4 |
| 1,50 | 16,4 | 12,8 | 14,3 |
| 2,00 | 16,0 | 13,3 | 12,0 |
| 3,00 | 12,2 | 13,8 | 14,4 |
| 4,00 | 8,0 | 12,5 | 14,7 |
| 6,00 | 3,7 | 8,4 | 9,3 |
| 8,00 | 2,0 | 4,2 | 5,5 |
| 10,00 | 1,7 | 3,4 | 4,3 |
| 12,00 | 1,6 | 2,8 | 3,2 |
| 14,00 | 0,9 | 2,1 | 2,8 |
| 24,00 | - | 1,0 | 1,3 |
| 28,00 | - | 0,6 | 0,6 |
| Concentrations plasmatiques observées après administration unique des comprimés 15, 20, 25 mg à 6 sujets sains (moyenne). | | | |

L'exemple du comprimé de 20mg est développé et comparé à la même dose dans les formes connues (tableau II).

Tableau II

|  | Poudre 20mg | Coprécipité 20mg | Comprimé 20 mg |
|---|---|---|---|
| Temps (h) | Concentration en ng/ml | Concentration en ng/ml | Concentration en ng/ml |
| 0,00 | 0,0 | 0,0 | 0,0 |
| 0,17 | 1,2 | 1,4 | 1,8 |
| 0,33 | 1,4 | 28,9 | 11,9 |
| 0,50 | 2,7 | 76,3 | 17,2 |
| 0,66 | 4,2 | 95,5 | 18,1 |
| 0,83 | 5,0 | 93,4 | 17,1 |
| 1,00 | 6,0 | 75,8 | 16,2 |
| 1,25 | 6,8 | 55,7 | 14,2 |
| 1,50 | 6,5 | 43,4 | 12,8 |
| 2,00 | 6,2 | 28,2 | 13,3 |
| 3,00 | 5,1 | 15,7 | 13,8 |
| 4,00 | 4,0 | 10,5 | 12,5 |
| 6,00 | 2,4 | 5,0 | 8,4 |
| 8,00 | 1,6 | 3,0 | 4,2 |
| 10,00 | 1,4 | 2,2 | 3,4 |
| 12,00 | 1,2 | 1,9 | 2,8 |
| 14,00 | 1,0 | 1,4 | 2,1 |
| 24,00 | 1,2 | 1,0 | 1,0 |
| 28,00 | - | - | 0,6 |
| Concentrations plasmatiques observées après administration orale unique de trois formes d'Oxodipine chez 6 sujets sains (moyenne). | | | |

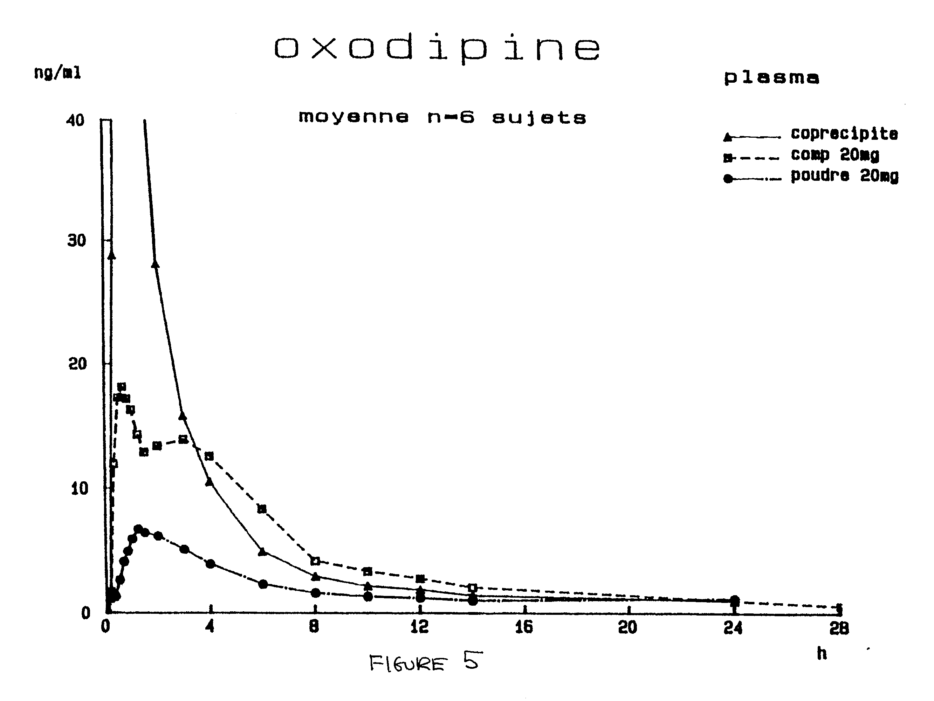

Cette comparaison souligne que les taux contrôlés atteints sont bien intermédiaires entre ceux relevant des deux formes connues (figure 5), et sont parfaitement régularisés pendant la période de 24 heures. On remarquera en particulier qu'en moins d'une heure le coprécipité induit une concentration sanguine de 95 ng/ml de sérum et que la figure 5 ne trace la courbe correspondante qu'à partir de 1H50 (43 ng/ml) pour des rasions de clarté du dessin.

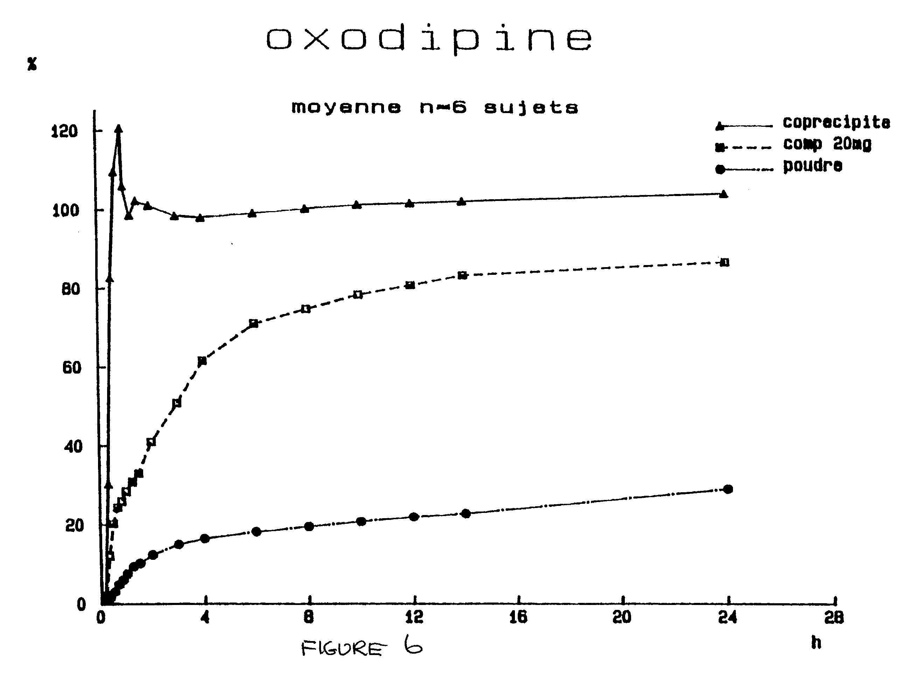

L'analyse des résultats confirme (figure 6) le bon contrôle de la dissolution-absorption du principe actif à partir de la forme comprimés.

Tableau III

| % DE DOSE ENTRES DANS L'ORGANISME APRES ABSORPTION ET PASSAGE HEPATIQUE | | | | | |
|---|---|---|---|---|---|
| TEMPS (h) | POUDRE 20 mg | COPRÉCIPITÉ | COMPRIME 15 mg | COMPRIME 20 mg | COMPRIME 25 mg |
| 0,00 | - | - | - | - | - |
| 0,17 | 2,5 | 32 | 2,4 | 2,1 | 5,9 |
| 0,33 | 1,6 | 60 | 12,6 | 12,1 | 15 |
| 0,50 | 4,5 | 75 | 26,2 | 20,3 | 23,5 |
| 0,66 | 6,6 | 87 | 32,7 | 24,5 | 26 |
| 0,83 | 7,0 | 93 | 35,5 | 26 | 28,1 |
| 1,00 | 9,3 | 96 | 38,9 | 31,9 | 28,3 |
| 1,25 | 11 | 96 | 46,4 | 31,0 | 30,5 |
| 1,50 | 11,4 | 97 | 50,2 | 33 | 32,8 |
| 2,00 | 14,0 | 97 | 59,0 | 41,1 | 39,2 |
| 3,00 | 16,6 | 100 | 68,7 | 51,0 | 48,1 |
| 4,00 | 18 | 100 | 72,7 | 61,7 | 53,7 |
| 6,00 | 19,8 | 100 | 74,5 | 71,1 | 61,0 |
| 8,00 | 21,2 | 100 | 75,6 | 74,8 | 63,8 |
| 10,00 | 22,6 | 100 | 77,2 | 78,5 | 66,6 |
| 12,00 | 23,8 | 100 | 79,2 | 81,1 | 68,2 |
| - | - | - | - | - | - |
| - | - | - | - | - | - |
| - | - | - | - | - | - |
| BIODIOPONIBILITE RELATIVE PAR RAPPORT A LA SOLUTION | 31 % | 100 | 86 % | 92 % | 88 % |

EP 0 412 877 A1

Les résultats indiqués dans le tableau III montrent que la biodisponibilité de chaque forme est totale pour le coprécipité (100%) faible pour la poudre d'oxodipine pure (31%) et excellente pour les comprimés (86 à 92%) au bout de 12 heures.

Ces résultats ont été confortés par ceux que la demanderesse a obtenu, avec d'autres dihydropyridines, et notamment la NIFEDIPINE.

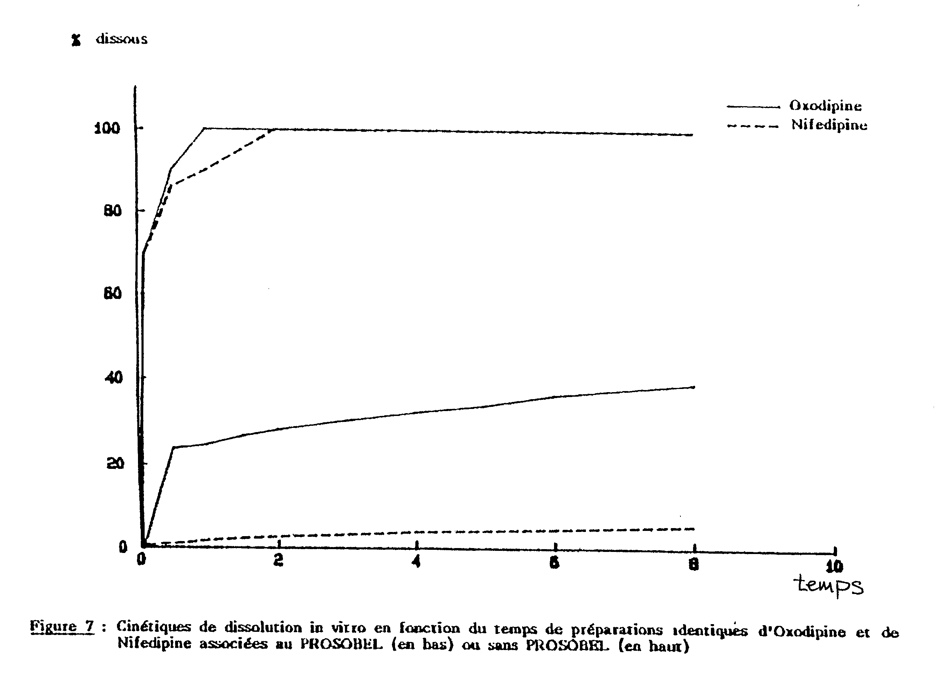

Le figure 7 montre le comportement parallèle entre l'oxodipine et la Nifédipine. Les cinétiques de dissolution, très rapides pour ces deux dihydropyridines (à l'état brut pour la Nifédipine, très soluble dans l'eau, et à l'état de coprécipité pour l'oxodipine) sont maîtrisées par l'adjonction de PROSOBEL, dans les mêmes formules qu'aux exemples 2 et 3.

On peut en conclure que le PROSOBEL, de façon très inattendue pour l'homme de métier, ralentit la dissolution en milieu aqueux, et permet l'obtention de formes pharmaceutiques originales et nouvelles, de dihydropyridines à biodisponibilité contrôlée.

Naturellement, les exemples donnés n'ont pas pour objet de limiter l'invention, celle-ci étant parfaitement applicable et efficace lorsque le mélange contenant le PROSOBEL est présenté sous forme de granules, capsules ou toute forme orale solide ou semi-solide tel qu'un gel muco-adhésif ou une pâte, ou même une suspension. Selon la forme considérée ou selon la solubilité aqueuse du principe actif mis en jeu, l'expérience a montré que l'adjonction de 10 à 70% de PROSOBEL permettait d'adapter la biodisponibilité du médicament aux taux plasmatiques souhaités.

## Revendications

1) Procédé de contrôle de la biodisponibilité d'un médicament oral, qui consiste à intégrer aux excipients inactifs 10 à 70% en poids de protéines naturelles.

2) Procédé selon la revendication 1, dans lequel les protéines naturelles sont extraites du lait.

3) Procédé selon l'une des revendications 1 ou 2, dans lequel les protéines naturelles sont du PROSOBEL.

4) Composition pharmaceutique à biodisponibilité contrôlée comprenant au moins un principe actif agissant comme inhibiteur calcique, des excipients usuels, et des protéines naturelles.

5) Composition pharmaceutique selon la revendication 4, dans laquelle l'inhibiteur calcique est une dihydropyridine.

6) Composition pharmaceutique selon l'une quelconque des revendications 4 ou 5, dans laquelle le principe actif est l'oxodipine.

7) Composition pharmaceutique selon l'une quelconque des revendications 4 ou 5 dans laquelle le principe actif est la Nifédipine.

8) Composition pharmaceutique selon l'une quelconque des revendications 4 à 7, dans laquelle les protéines naturelles sont extraites du lait.

9) Composition pharmaceutique selon l'une quelconque des revendications 4 à 8, dans laquelle les protéines sont du PROSOBEL.

10) Composition pharmaceutique selon l'une quelconque des revendications 4,5,6,8 ou 9 caractérisée en ce qu'elle contient de l'oxodipine et du PROSOBEL L60.

11) Composition pharmaceutique selon l'une quelconque des revendications 4,5,6,8 ou 9, caractérisée en ce qu'elle contient de l'oxodipine, et du PROSOBEL L85.

12) Composition pharmaceutique selon l'une quelconque des revendications 4,5,7,8, ou 9, caractérisée en ce qu'elle contient de la Nifédipine et du PROSOBEL L60.

13) Composition pharmaceutique selon l'une quelconque des revendications 4,5,7, 8 ou 9, caractérisée en ce qu'elle contient de la Nifédipine et du PROSOBEL L85.

14) Composition pharmaceutique selon l'une quelconque des revendications 4 à 13, caractérisée en ce qu'elle est contenue dans une gélule constituant une dose unitaire quotidienne.

15) Composition pharmaceutique selon l'une quelconque des revendications 4 à 13, caractérisée en ce qu'elle est sous forme de comprimé contenant la dose unitaire quotidienne.

16) Composition pharmaceutique selon la revendication 15, caractérisée en ce que le comprimé est bicouches, l'une des couches contenant la dose de charges à libération rapide et l'autre couche étant à libération lente.

Revendications pour les Etats contractants suivants: ES, GR

1) Procédé de préparation d'une composition pharmaceutique à biodisponibilité controlée du principe actif, qui consiste à intégrer à des excipients usuels 10 à 70 % en poids de protéines naturelles.

2) Procédé selon la revendication 1, dans lequel les protéines naturelles sont extraites du lait.

3) Procédé selon l'une des revendications 1 ou 2, dans lequel les protéines naturelles sont du PROSOBEL.

4) Procédé de préparation selon l'une des revendications 1 à 3, caractérisé en ce que la composition pharmaceutique comprend au moins un principe actif agissant comme inhibiteur calcique.

5) Procédé de préparation selon la revendication 4, caractérisé en ce que l'inhibiteur calcique est une dihydropyridine.

6) Procédé de préparation selon l'une des revendications 4 ou 5, caractérisé en ce que le principe actif est l'oxodipine.

7) Procédé de préparation selon l'une des revendications 4 ou 5, caractérisé en ce que le principe actif est la nifedipine.

8) Procédé de préparation selon l'une des revendications 4, 5, 6 ou 7 caractérisé en ce que la composition pharmaceutique contient du PROSOBEL L60.

9) Procédé de préparation selon l'une des revendications 4, 5, 6 ou 7 caractérisé en ce que la composition pharmaceutique contient du PROSOBEL L85.

Figure 1 : Cinétique de dissolution intestinale in vivo chez l'homme de deux préparations d'oxodipine. (Résultats moyens sur 6 sujets)

# OXODIPINE (10MG)

FIGURE 2

EP 0 412 877 A1

FIGURE 3 :  CINETIQUES MOYENNES

**DISSOUS(%)**

**non DISSOUS(%)**

X Absorbés in vivo

CINETIQUES DE DISSOLUTION IN VIVO ETABLIES EN FONCTION DE LA SOLUTION SOLIDE ( ■ ) DES FORMES F ( ✳ ) ET G ( ● ) SELON EXEMPLE 2 ET DE LA POUDRE ( ◇ ). LA REPRESENTATION DES FRACTIONS TOTALES DISSOUTES ET NON DISSOUTES FIGURE EN HAUT A DROITE POUR CHAQUE FORMES.

EP 0 412 877 A1

# oxodipine

### moyenne n=6 sujets

FIGURE 4

FIGURE 5

# oxodipine

**moyenne n=6 sujets**

FIGURE 6

**X dissous**

Oxodipine

Nifedipine

temps

Figure 7 : Cinétiques de dissolution in vitro en fonction du temps de préparations identiques d'Oxodipine et de Nifedipine associées au PROSOBEL (en bas) ou sans PROSOBEL (en haut)

EP 0 412 877 A1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | GB-A-2159407 (TAKADA SEIYAKU KABUSHIKI KAISHA) <br> * le document en entier * | 1-2, <br> 4-5, <br> 7-8, <br> 14-15 | A61K9/20 <br> A61K47/42 <br> A61K31/44 |
| X | PATENT ABSTRACTS OF JAPAN <br> vol. 10, no. 131 (C-346)(2188) 15 mai 1986, <br> & JP-A-60 255719 (TAKADA SEIYAKU K.K.) 17 <br> décembre 1985, <br> * le document en entier * | 1-2, <br> 4-5, <br> 7-8, <br> 14-15 | |
| X | US-A-3184386 (DOUGLAS STEPHENSON ET AL.) <br> * colonne 3, lignes 12 - 21; revendications 1-9 * | 1-2, <br> 14-16 | |
| X <br> A | US-A-4670251 (JOHN F. BLANCO) <br> * revendications 1-25 * | 1-2 <br> 3-16 | |
| X <br><br><br> A | EP-A-282020 (NISSHIN FLOUR MILLING CO.,LTD.) <br> * page 3, lignes 31 - 34 * <br> * page 3, ligne 56 - page 4, ligne 8; revendications 1-10 * | 1-2 <br><br><br> 3-16 | **DOMAINES TECHNIQUES RECHERCHES (Int. Cl.5)** <br><br> A61K |

Le présent rapport a été établi pour toutes les revendications

| Lieu de la recherche | Date d'achèvement de la recherche | Examinateur |
|---|---|---|
| BERLIN | 05 NOVEMBRE 1990 | SIATOU, E. |